# EUROPÄISCHE PATENTANMELDUNG

(11) **EP 1 285 631 A2**
(43) Veröffentlichungstag der Anmeldung: **26.02.2003**
(21) Anmeldenummer: 02405590.7
(22) Anmeldetag: 11.07.2002
(51) Int. Cl.: A61B 17/28, A61F 9/007

(54) **Mikrochirurgisches Instrument**

(30) Priorität: 23.08.2001 US 935869
(71) Anmelder: Alcon Grieshaber AG, 8203 Schaffhausen (CH)
(72) Erfinder: Arumi, Jose Garcia Dr. MD., 08017 Barcelona (ES); Maag, Werner, 8750 Glarus (CH); Jud, Oliver, 8224 Löhningen (CH)
(74) Vertreter: Althoff, Gerhard

(57) **Zusammenfassung**

Mit vorliegender Erfindung wird ein ophthalmologisches Instrument für die Netzhautchirurgie vorgeschlagen, welches beim chirurgischen Eingriff zum Greifen sowie klemmfreien und freibeweglichen Halten feiner Strukturen in Form von Blutgefässen oder dergleichen ausgebildet ist.

Das Instrument hat ein als Handgriff ausgebildetes Gehäuse mit daran angeordneter Funktionseinheit, welche eine darin gelagerte und als Hohlnadel ausgebildete Sonde (46) und eine koaxial darin gelagerte Stange umfasst, wobei die Stange am vorderen freien Ende mit einem als Greifelement ausgebildeten Kopfstück (50) versehen ist. Die Stange hat zwei am vorderen Ende jeweils mit einer Aussparung versehene Arme, wobei die beiden Arme relativ zueinander spreizbar und entgegen der federelastischen Vorspannung zum formschlüssigen Schliessen einer entsprechend der beiden Aussparungen ausgebildeten Ausnehmung (55) zusammendrückbar sind.

## Beschreibung

Die Erfindung bezieht sich auf ein mikrochirurgisches Instrument, insbesondere auf ein ophthalmologisches Instrument für die Netzhautchirurgie, bestehend aus einem als Handgriff ausgebildeten und mit Mitteln zum Betätigen eines Schiebebolzens versehenen Gehäuse, einer daran angeordneten Funktionseinheit mit darin gelagertem und mit dem Schiebebolzen zusammenwirkenden Stellglied sowie einer damit wirkverbundenen röhrchenförmigen Sonde, welche mindestens zur Aufnahme einer in axialer Richtung orientierten und am vorderen Ende mit einem Kopfstück versehenen Stange ausgebildet ist.

Die vorliegende Erfindung befasst sich mit dem Problem der chirurgischen Behandlung von retinalen Erkrankungen, beispielsweise durch Hypertonie oder vaskuläre Veränderungen bewirkte Erkrankungen, wodurch im Bereich der sich kreuzenden und an dieser Stelle von einer weitgehend transparenten Haut umhüllten Arterie und Vene ein sogenannter Venenastverschluss entstehen und dabei die Vene von der darauf liegenden Arterie zusammengedrückt beziehungsweise abgeklemmt werden kann.

Versuche haben gezeigt, dass durch Auftrennen beziehungsweise durch Entfernen der Hauthülle mittels eines an sich bekannten ophthalmologischen Instruments derartige Venenastverschlüsse weitgehend eliminiert oder verhindert werden können. Bei diesem mikrochirurgischen Eingriff ist es jedoch zweckmässig, dass während des Schneid- oder Trennvorgangs gleichzeitig durch eine geringe Zugbewegung die Arterie ohne jegliche Klemmwirkung von der Vene entfernt wird.

Der Erfindung liegt die Aufgabe zugrunde, ein ophthalmologisches Instrument zu schaffen, welches zum Greifen und freibeweglichen Halten feiner Strukturen in Form von Blutgefässen oder dergleichen ausgebildet ist.

Das erfindungsgemässe Instrument ist dadurch gekennzeichnet, dass die Stange ausgehend von dem Kopfstück zwei in axialer Richtung durch einen Spalt getrennte und zur Erreichung einer federelastischen Vorspannung relativ zueinander spreizbare sowie am vorderen Ende jeweils mit einer Aussparung versehene und entgegen der Vorspannung zusammendrückbare Arme aufweist, bei welchen die einander zugewandten Aussparungen in der Schliessstellung eine quer zur Längsachse der Stange orientierte und zum klemmfreien sowie freibeweglichen Halten feiner Strukturen ausgebildete Ausnehmung bilden.

Weitere Merkmale und Ausführungsbeispiele der Erfindung ergeben sich aus der nachstehenden Beschreibung in Verbindung mit der Zeichnung und den einzelnen Patentansprüchen.

Die Erfindung wird nachstehend anhand der Zeichnung beschrieben. Es zeigt:
- **Fig.1**: ein in grösserem Massstab dargestelltes Auge eines Lebewesens mit einer in den Hohlraum des Glaskörpers eingeführten und mit einem ophthalmologischen Instrument wirkverbundenen Sonde;
- **Fig.2**: ein in Fig.1 durch einen Kreis K bezeichnetes Teilstück des räumlich dargestellten Blutgefäss-Systems mit einem damit in Eingriff stehenden Greifelement des Instruments;
- **Fig.3**: das mit einer Funktionseinheit sowie der daran angeordneten Sonde und dem darin angeordneten Greifelement versehene Instrument in räumlicher Ansicht;
- **Fig.4**: die in grösserem Massstab sowie im Schnitt dargestellte Funktionseinheit mit der Sonde sowie einer darin gelagerten Stange mit schematisch und geöffnet dargestelltem Greifelement;
- **Fig.5**: die mit den einzelnen Elementen versehene Funktionseinheit gemäss Fig.4 mit geschlossen dargestelltem Greifelement;
- **Fig.6**: ein in grösserem Massstab sowie im Schnitt dargestelltes Teilstück der röhrchenförmigen Sonde mit einer ersten Variante des an der Stange angeformten Greifelements in geöffneter Stellung;
- **Fig.7**: die gemäss Fig.6 in Draufsicht dargestellte Sonde mit der Stange und dem Greifelement;
- **Fig.8**: die Sonde mit der Stange und dem in geschlossener Stellung dargestellten Greifelement gemäss Fig.6;
- **Fig.9**: das gemäss der in Fig.8 eingezeichneten Pfeilrichtung A in Stirnansicht dargestellte und in der Sonde angeordnete Greifelement mit der Stange;
- **Fig.10**: die gemäss der in Fig.8 eingezeichneten Linie B-B im Schnitt dargestellte Sonde mit der Stange und dem Greifelement;
- **Fig.11**: eine Variante der in Ansicht dargestellten Stange mit angeformtem und geöffnet dargestellten Greifelement gemäss Fig.6;
- **Fig.12**: die gemäss der in Fig.11 eingezeichneten Linie C-C im Schnitt dargestellte Stange;
- **Fig.13**: die im Schnitt dargestellte Sonde gemäss Fig.6 mit einer zweiten Variante des an der Stange angeformten Greifelements in geöffneter Stellung;
- **Fig.14**: die Sonde mit dem geschlossenen Greifelement gemäss Fig.13;
- **Fig.15**: die im Schnitt dargestellte Sonde gemäss Fig.6 mit einer weiteren Variante des an der Stange angeformten Greifelements in geöffneter Stellung;
- **Fig.16**: die Sonde mit dem geschlossenen Greifelement gemäss Fig.15;
- **Fig.17**: eine etwa in räumlicher Ansicht und teilweise im Schnitt dargestellte Variante der mit den einzelnen Elementen versehenen Funktionseinheit für das ophthalmologische Instrument gemäss Fig.3;
- **Fig.18**: ein gemäss der in Fig.17 eingezeichneten Linie D-D im Schnitt dargestelltes Teilstück der Sonde mit der darin angeordneten Stange sowie zugeordnetem Lichtleiter;
- **Fig.19**: ein gemäss der in Fig.17 eingezeichneten Linie E-E im Schnitt dargestellte Teilstück der Sonde mit der Stange und dem zugeordneten Lichtleiter; und
- **Fig.20**: das gemäss der in Fig.17 eingezeichneten Pfeilrichtung F in Draufsicht dargestellte vordere Teilstück der Sonde mit dem Lichtleiter und dem als Greifelement ausgebildeten Kopfstück.

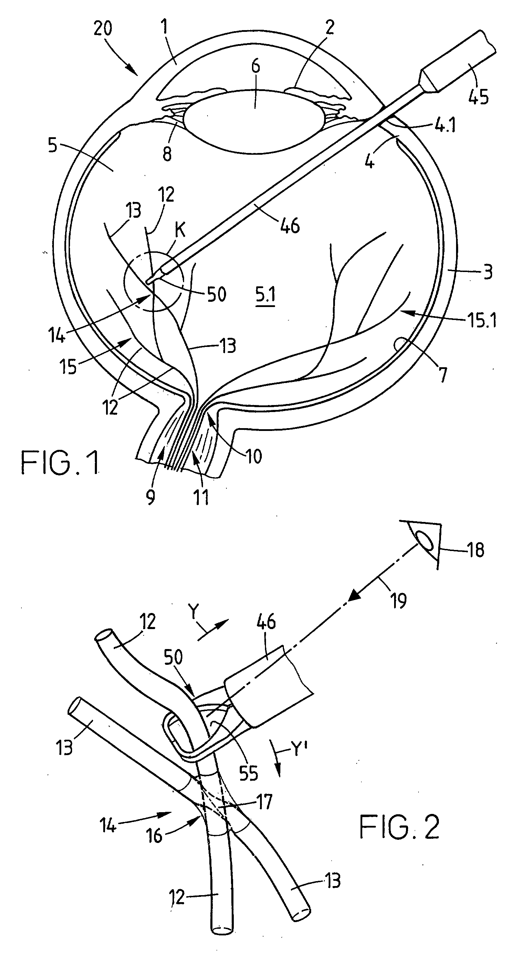

In Fig.1 ist zur Verdeutlichung der Erfindung als allgemeine Übersicht ein Auge 20 in grösserem Massstab sowie als schematischer Horizontalschnitt dargestellt und man erkennt die Hornhaut 1 (CORNEA), die Regenbogenhaut 2 (IRIS),die Lederhaut 3 (Sklera) mit der Pars plana 4, den in der Gesamtheit mit 5 bezeichneten Glaskörper mit dem Glaskörperhohlraum 5.1, die Linse 6 (LENS), die Netzhaut 7 (RETINA) sowie die Strahlenbänder 8 (ZONULA). Im Bereich des Augenhintergrunds erkennt man die etwa scheibenförmige Papille 10 (OPTIC DISK), in welcher sich die Nervenfasern der Netzhaut 7 sammeln und als Sehnervenbündel 9 (OPTICUS) das Auge 20 verlassen.

Das von dem Sehnervenbündel 9 umgebene und in der Gesamtheit mit 11 bezeichnete zentrale Arterien- und Venensystem verzweigt sich in der Papille 10 in mehrere Äste, welche zusammen die beiden Blutgefäss-Systeme 15 und 15.1 bilden. Das in Fig.1 schematisch dargestellte Blutgefäss-System 15 hat die mit 12 bezeichneten Arterien sowie die mit 13 bezeichneten Venen, welche beispielsweise im Bereich 14 kreuzend übereinander liegen und durch eine relativ dünne, weitgehend transparente Haut (Fig.2) miteinander verbunden sind. Die Arterien 12 und Venen 13 des einzelnen Blutgefäss-Systems 15 beziehungsweise 15.1 haben jeweils einen Aussendurchmesser von etwa 0,1 bis 0,15 mm.

Weiterhin erkennt man in Fig.1 eine als längliche Hohlnadel ausgebildete Sonde 46, welche durch eine in der Pars plana 4 vorgesehene Inzision 4.1 in den Glaskörperhohlraum 5.1 eingeführt ist. Die als Hohlnadel ausgebildete und in den Glaskörperhohlraum 5.1 einführbare Sonde 46 hat beispielsweise einen Aussendurchmesser von etwa 1,0 mm und einen Innendurchmesser von etwa 0,8 mm. In der röhrchenförmigen Sonde 46 ist eine in axialer Richtung orientierte Stange angeordnet, welche am vorderen aus der Sonde 46 ragenden Ende mit einem zum Greifen und Halten feiner Strukturen ausgebildeten Kopfstück 50 versehen ist. Das als Greifelement ausgebildete Kopfstück 50 sowie weitere Ausgestaltungen desselben wird/werden nachstehend noch im einzelnen beschrieben.

In Fig.2 ist der in Fig.1 durch einen Kreis K bezeichnete Bereich 14 des einen Blutgefäss-Systems 15 räumlich sowie in grösserem Massstab dargestellt und man erkennt ein Teilstück der von dem schematisch dargestellten und aus der Sonde 46 ragenden Kopfstück 50 erfassten Arterie 12 sowie die darunter liegende Vene 13. Die Arterie 12 sowie die Vene 13 sind im Bereich 14, wie in Fig.2 schematisch dargestellt, durch eine weitgehend transparente Hauthülle 16 umgeben und dadurch miteinander verbunden. Wie vorstehend erwähnt, kann im Bereich 14 der sich kreuzenden Gefässe ein sogenannter Venenastverschluss entstehen, bei welchem die Vene 13 von der darüber liegenden Arterie 12 zusammengedrückt beziehungsweise abgeklemmt wird. Durch Auftrennen der Hauthülle 16, beispielsweise gemäss der in Fig.2 schematisch dargestellten Linie 17 beziehungsweise durch Entfernen der Hauthülle 16 mittels eines an sich bekannten ophthalmologischen Instruments können derartige Venenastverschlüsse weitgehend eliminiert oder verhindert werden. Bei diesem mikrochirurgischen Eingriff ist es jedoch auch erforderlich, dass während des Schneid- oder Trennvorgangs gleichzeitig das Instrument mit dem Kopfstück 50 durch eine in Pfeilrichtung Y und Y' orientierte Bewegung die Arterie 12 ohne jegliche Klemmwirkung von der Vene 13 entfernt wird. Das mit einer entsprechend ausgebildeten Ausnehmung 55 versehene Kopfstück 50 ist vorzugsweise so ausgebildet, dass die in der Ausnehmung 55 gehaltene Arterie 12 von dem in Fig.2 schematisch dargestellten Auge 18 des Ophthalmologen während des operativen Eingriffs visuell gemäss der theoretischen Achse 19 beobachtet werden kann.

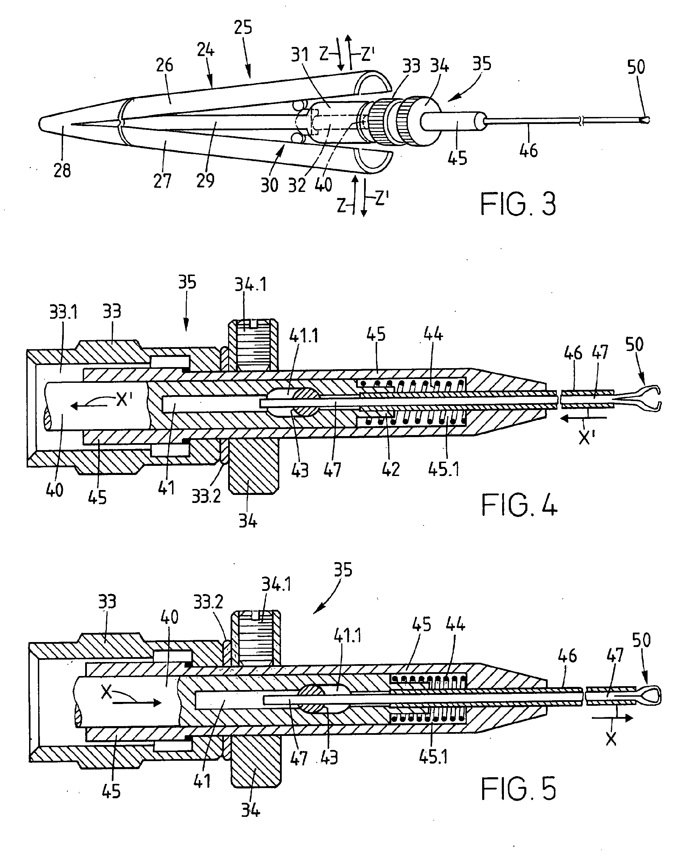

Fig.3 zeigt als Ausführungsbeispiel ein räumlich sowie in Ansicht dargestelltes ophthalmologisches Instrument 25, welches speziell zum Greifen und Halten feiner Strukturen jeweils mit dem entsprechend als Greifelement ausgebildeten Kopfstück 50 versehen ist. Das Instrument 25 umfasst im wesentlichen ein als Handgriff ausgebildetes Gehäuse 24 mit zwei halbschalenförmigen Gehäuseteilen 26 und 27, welche am hinteren Ende in einer Verschlusskappe 28 fest miteinander verbunden sind. Zwischen den beiden Gehäuseteilen 26 und 27 ist ein mit einer Spreizvorrichtung 30 wirkverbundener Tragarm 29 sowie ein zylindrisches Führungsstück 31 angeordnet. Der Tragarm 29 ist zur aufschraubbaren Befestigung einer mit einem Stellglied 40 und der Sonde 46 versehenen Funktionseinheit 35 (Fig.4,5) ausgebildet, wobei das Stellglied 40 mit einem in dem Führungsstück 31 gelagerten Schiebebolzen 32 sowie mit der Spreizvorrichtung 30 wirkverbunden ist.

Bei dem in Fig.3 als Ausführungsbeispiel dargestellten Instrument 25 ist durch die in Pfeilrichtung Z orientierte Bewegung der beiden Gehäuseteile 26,27 der in dem Führungsstück 31 gelagerte Schiebebolzen 32 mittels der Spreizvorrichtung 30 in axialer Richtung verschiebbar. Bei der in Pfeilrichtung Z' orientierten Bewegung der beiden Gehäuseteile 26,27 ist der Schiebebolzen 32 durch die Rückstellkraft einer in der Funktionseinheit 35 (Fig.4,5) angeordneten Druckfeder entgegengesetzt in axialer Richtung verschiebbar. Das Instrument 25 mit den beiden Gehäuseteilen 26,27 sowie die dazwischen angeordnete Spreizvorrichtung 30 mit dem Führungsstück 31 und dem Schiebebolzen 32 sind nicht Gegenstand vorliegender Erfindung und werden deshalb nicht näher beschrieben.

Fig.4 zeigt die im Schnitt sowie in grösserem Massstab dargestellte Funktionseinheit 35 und man erkennt die mit einer Ausnehmung 33.1 versehene Überwurfmutter 33 sowie eine darin gelagerte Führungshülse 45 mit am äusseren Durchmesser angeordnetem Zwischenring 33.2 und Stellring 34. Der Stellring 34 ist mit einem eingeschraubten Gewindestift 34.1 an der Führungshülse 45 gehalten, so dass die einzelnen Teile eine Baueinheit bilden. In einer Ausnehmung 45.1 der Führungshülse 45 ist das in nicht näher dargestellter Weise mit dem Schiebebolzen 32 (Fig.3) zusammenwirkende Stellglied 40 angeordnet, welches an dem einen Ende mit einem abgesetzt ausgebildeten zylindrischen Teilstück 42 versehen ist. An dem zylindrischen Teilstück 42 ist eine in der Ausnehmung 45.1 der Führungshülse 45 angeordnete und entsprechend abgestützte Druckfeder 44 gelagert.

Das Stellglied 40 hat eine Sacklochbohrung 41 sowie eine damit korrespondierende und mit 41.1 bezeichnete Ausnehmung, welche das Stellglied 40 radial durchdringt. Die Sacklochbohrung 41 ist zur Aufnahme und Lagerung der als Hohlnadel ausgebildeten Sonde 46 ausgebildet, wobei in der röhrchenförmigen Sonde 46 eine längliche Stange 47 gelagert ist. Die Stange 47 ist an dem vorderen freien Ende mit dem Kopfstück 50, vorzugsweise angeformten Kopfstück 50 versehen. Die röhrchenförmige Sonde 46 ist in nicht dargestellter Weise, beispielsweise durch eine Kleb-, Schweiss- oder Lötverbindung mit dem Stellglied 40 wirkverbunden. Die Stange 47 ist an dem in der Sonde 46 gelagerten Ende durch mindestens einen radial in die Führungshülse 45 eingeschraubten Gewindestift 43 gegen axiales Verschieben gesichert. An dem anderen, aus der röhrchenförmigen Sonde 46 ragenden Ende ist an der Stange 47 das zum Greifen und Halten feiner Strukturen ausgebildete Kopfstück 50 angeordnet. In Fig.4 ist das Kopfstück 50 infolge der in Pfeilrichtung X' zurückgezogenen Sonde 46 in weitgehend geöffneter Stellung dargestellt.

Fig.5 zeigt die vorstehend in Verbindung mit Fig.4 beschriebene Funktionseinheit 35 mit den einzelnen Elementen. Abweichend von Fig.4 ist in Fig.5 das Stellglied 40 entgegen der Rückstellkraft der Druckfeder 44 relativ zu der durch den Gewindestift 43 mit der Führungshülse 45 gesicherten Stange 47 gemäss Pfeilrichtung X in axialer Richtung verschoben. In dieser Position ist das Kopfstück 50 von der in axialer Richtung verschobenen Sonde 46 geschlossen. Die in axialer Richtung relativ zu dem an der Stange 47 angeordneten Kopfstück 50 orientierte Bewegung der röhrchenförmigen Sonde 46 wird mittels des in Fig.3 als Ausführungsbeispiel dargestellten und von dem Ophthalmologen betätigten Instruments 25 erreicht.

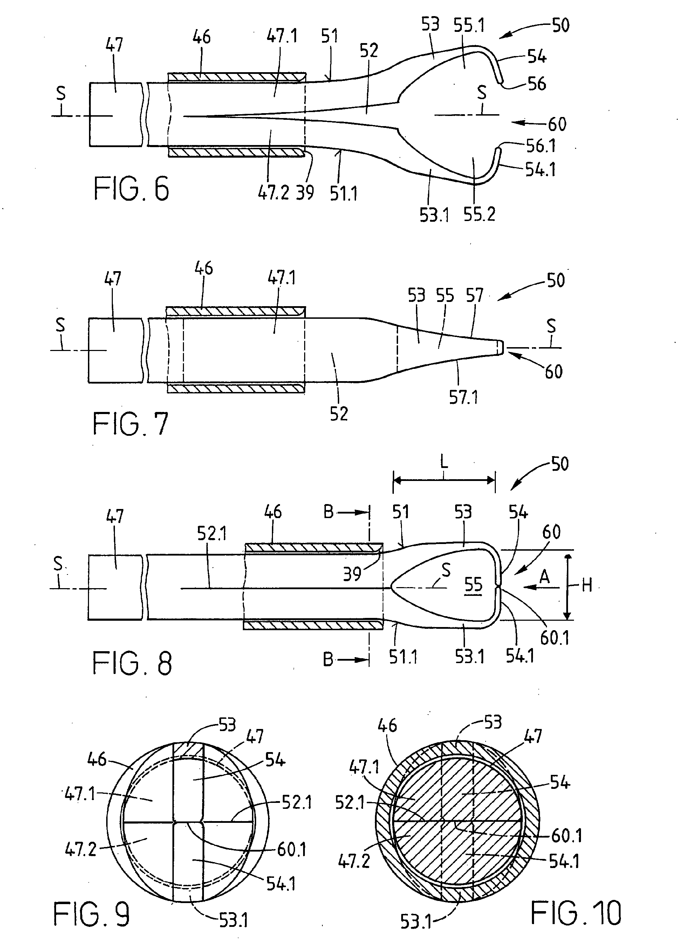

Nachstehend werden anhand der Figuren 6 bis 16 einzelne Ausführungsbeispiele der in der röhrchenförmigen Sonde 46 angeordneten Stange zusammen mit dem beispielsweise angeformten und als Greifelement ausgebildeten Kopfstück beschrieben, wobei nachstehend die Stange allgemein mit 47 und das Kopfstück allgemein mit 50 bezeichnet wird.

In Fig.6 bis Fig.8 ist die röhrchenförmige Sonde 46 sowie die koaxial darin angeordnete Stange 47 in grösserem Massstab sowie im Schnitt dargestellt. Bei diesem Ausführungsbeispiel ist die Stange 47 als länglicher, einstückiger Zylinderkörper ausgebildet und an dem vorderen aus der Sonde 46 ragenden Teilstück mit dem als erste Variante ausgebildeten und in geöffneter Stellung dargestellten Kopfstück 50 versehen. Die Sonde 46 ist am vorderen Ende innenseitig mit einer Abrundung 39 versehen.

Zur formgebenden Ausgestaltung des Kopfstücks 50 ist die Stange 47 ausgehend von der Stirnseite 60 mit geeigneten, nicht dargestellten Mitteln in axialer Richtung eingeschnitten und im Bereich des Einschnitts oder Spalts 52 in zwei in axialer Richtung orientierte Teilstücke oder Arme 47.1 und 47.2 unterteilt. Zur Erreichung einer federelastischen Vorspannung sind die beiden Arme 47.1 und 47.2 relativ zueinander beziehungsweise relativ zu einer Symmetrieachse S-S gespreizt oder aufgebogen, so dass zwischen den beiden Armen 47.1 und 47.2 der in Längsrichtung der Stange 47 orientierte Spalt 52 entsteht. Die beiden Arme 47.1 und 47.2 sind in dieser Stellung an den gegenüberliegend zueinander angeordneten Seiten jeweils als eine in Richtung des Kopfstücks 50 schräg ansteigende Gleitfläche 51 und 51.1 ausgebildet (Fig6). Die Gleitflächen 51 und 51.1 sind jeweils kontinuierlich geradlinig oder bogenförmig ansteigend ausgebildet.

Im vorderen Bereich ist der eine Arm 47.1 mit einer ersten Aussparung 55.1 versehen, welche durch eine innenseitig etwa bogenförmig ausgebildete erste Wand 53 sowie durch einen daran angeformten Schenkel 54 stirnseitig begrenzt ist. Der andere Arm 47.2 ist mit einer zweiten Aussparung 55.2 versehen, welche durch eine innenseitig etwa bogenförmig ausgebildete zweite Wand 53.1 sowie durch einen daran angeformten Schenkel 54.1 stirnseitig begrenzt ist. Die beiden Schenkel 54 und 54.1 sind an den einander zugewandten Enden jeweils mit einer Kante 56 und 56.1 versehen. In geschlossenem Zustand sind die beiden Kanten 56 und 56.1 etwa quer zur Symmetrieachse S-S orientiert formschlüssig gegeneinander gedrückt sind (Fig.8) und bilden eine mit 60.1 bezeichnete Trennfuge.

Fig.7 zeigt ein Teilstück der röhrchenförmigen Sonde 46 sowie die darin gelagerte Stange 47 mit dem angeformten und in Draufsicht dargestellten Kopfstück 50. Das von der quer zur Symmetrieachse S-S orientierten Ausnehmung 55 durchdrungene Kopfstück 50 ist ausgehend von der Stirnseite 60 in Richtung des zylindrischen Teilstücks der Stange 47 konisch erweiternd ausgebildet, wobei die beiden gegenüberliegenden Seitenwände 57 und 57.1 der beiden Wände 53 und 53.1 entweder geradlinig oder bogenförmig ausgebildet sind.

Fig.8 zeigt die Sonde 46 mit der Stange 47 und dem angeformten und in dieser Stellung geschlossen dargestellten Kopfstück 50 und der Ausnehmung 55. Die in axialer Richtung der Symmetrieachse S-S orientierte lichte Länge L der aus den beiden etwa bogenförmigen Aussparungen 55.1 und 55.2 gebildeten Ausnehmung 55 ist grösser als die quer dazu orientierte lichte Breite H der Ausnehmung 55. In der geschlossenen Stellung des Kopfstücks 50 sind die einander zugewandten Innenkanten (nicht bezeichnet) der Arme 47.1 und 47.2 derart formschlüssig gegeneinander gedrückt, dass der Spalt 52 (Fig.6) als Trennfuge 52.1 erkennbar ist. Die beiden Schenkel 54 und 54.1 bilden dabei an der Stirnseite 60 die mit 60.1 bezeichnete Trennfuge.

In Fig.9 ist das Kopfstück 50 gemäss der in Fig.8 eingezeichneten Pfeilrichtung A in Ansicht sowie in grösserem Massstab dargestellt und man erkennt den teilweise aufgebrochen und im Profilquerschnitt dargestellten ersten Schenkel 54 sowie den gegenüberliegenden und mit 54.1 bezeichneten zweiten Schenkel, welche an den beiden Armen 47.1 und 47.2 der Stange 47 angeformt sind (Fig.6). In der Schliessstellung sind die beiden Schenkel 54 und 54.1 mit den korrespondierend einander zugewandten Kanten 56 und 56.1 entlang der Trennfuge 60.1 formschlüssig gegeneinander gedrückt. Weiterhin erkennt man in Fig.9 die in der röhrchenförmigen Sonde 46 angeordnete zylindrische Stange 47 mit der von dem Spalt 52 gebildeten Trennfuge 52.1.

Fig.10 zeigt einen Schnitt gemäss der in Fig.8 eingezeichneten Linie B-B und man erkennt die röhrchenförmige Sonde 46, die koaxial darin angeordnete Stange 47 mit den beiden durch den Spalt 52 getrennten Armen 47.1 und 47.2 sowie die beiden Schenkel 54 und 54.1, welche im Bereich der Trennfuge 60.1 formschlüssig gegeneinander gedrückt sind.

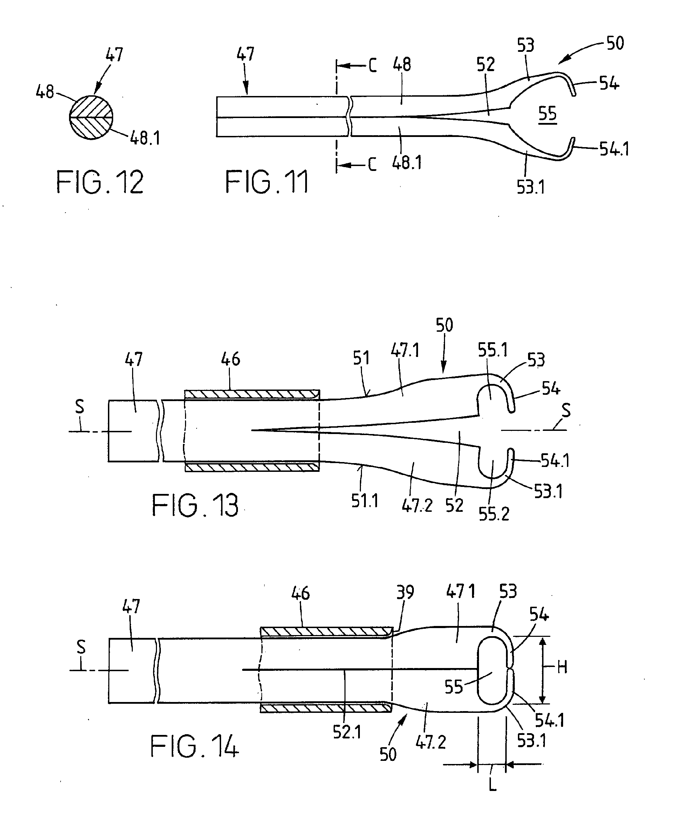

In Fig.11 ist eine weitere Ausgestaltung der Stange 47 mit dem Kopfstück 50 in Ansicht dargestellt, wobei in Fig.12 die Stange 47 gemäss der in Fig.11 eingezeichneten Schnittlinie C-C im Profilquerschnitt dargestellt ist. Das an dem vorderen Ende der Stange 47 angeformte Kopfstück 50 mit den Teilen 53 und 53.1 sowie 54 und 54.1 ist analog dem vorstehend in Verbindung mit Fig.6 bis Fig.8 beschriebenen Kopfstück 50 ausgebildet. Abweichend von dem Ausführungsbeispiel (Fig.6 bis Fig.8) umfasst die Stange 47 gemäss Fig.11 und Fig.12 zwei im Profilquerschnitt halbkreisförmig ausgebildete und mit 48 und 48.1 bezeichnete Teilstücke. Die beiden Teilstücke 48 und 48.1 sind von dem hinteren Ende in Richtung des Kopfstücks 50 beispielsweise durch eine Laserschweissung miteinander verbunden. Im vorderen Bereich sind die beiden halbkreisförmigen Teilstücke als Arme 48 und 48.1 durch den in axialer Richtung orientierten Spalt 52 getrennt und zur Erreichung einer federelastischen Vorspannung relativ zueinander gespreizt.

Fig.13 zeigt die in grösserem Massstab sowie im Schnitt dargestellte röhrchenförmige Sonde 46 mit der koaxial darin angeordneten Stange 47. Am vorderen Ende ist die Stange 47 mit einem als zweite Variante ausgebildeten und in geöffneter Stellung dargestellten Kopfstück 50 versehen. Die Stange 47 ist durch den Spalt 52 in zwei in axialer Richtung orientierte Arme 47.1 und 47.2 unterteilt sowie an den gegenüberliegend zueinander angeordneten Seiten jeweils mit einer in Richtung des Kopfstücks 50 ansteigenden Gleitfläche 51 und 51.1 versehen. Zur Erreichung der federelastischen Vorspannung sind die beiden Arme 47.1 und 47.2 relativ zueinander beziehungsweise relativ zu der Symmetrieachse S-S gespreizt oder aufgebogen. Im vorderen Bereich ist der eine Arm 47.1 mit einer ersten Aussparung 55.1 versehen, welche durch eine innenseitig kreisbogenförmig ausgebildete erste Wand 53 sowie durch einen daran angeformten Schenkel 54 stirnseitig begrenzt ist. Der andere Arm 47.2 ist mit einer zweiten Aussparung 55.2 versehen, welche durch eine innenseitig kreisbogenförmige zweite Wand 53.1 sowie durch einen daran angeformten Schenkel 54.1 stirnseitig begrenzt ist. Die beiden Aussparungen 55.1 und 55.2 bilden in geschlossenem Zustand des Kopfstücks 50 die Ausnehmung 55.

Fig.14 zeigt die Sonde 46 mit der koaxial darin angeordneten Stange 47 sowie das daran angeformte Kopfstück 50 mit der Ausnehmung 55. In der geschlossenen Stellung (Fig.14) sind die beiden stirnseitigen Schenkel 54 und 54.1 an den einander zugewandten Enden (nicht bezeichnet) formschlüssig gegeneinander gedrückt. Bei dieser Variante ist die in axialer Richtung des Kopfstücks 50 orientierte lichte Länge **L** der Ausnehmung 55 kleiner als die quer dazu orientierte lichte Breite **H** ausgebildet.

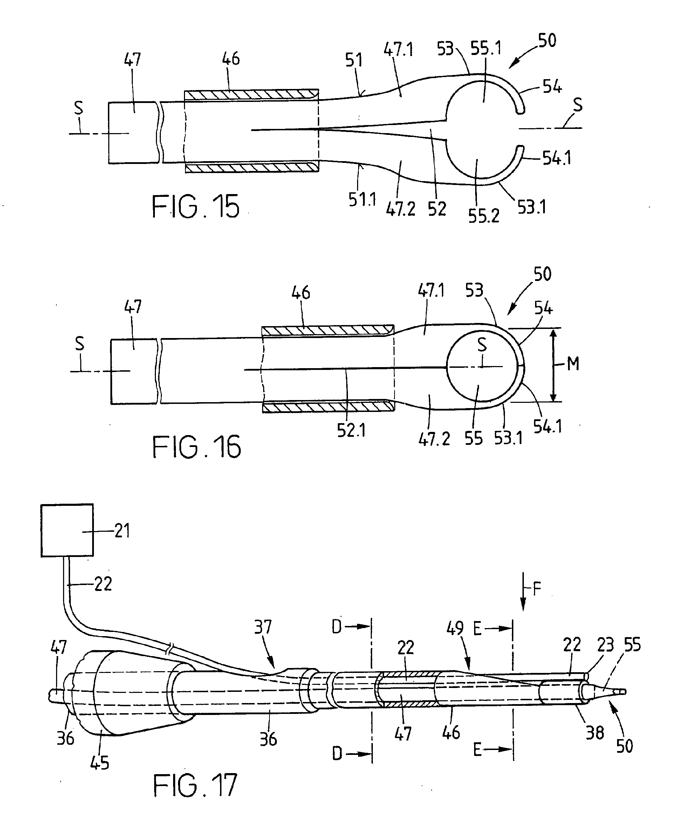

In Fig.15 und Fig.16 ist als dritte Variante die in der röhrchenförmigen Sonde 46 angeordnete und mit den beiden durch den Spalt 52 in zwei in axialer Richtung orientierte Arme 47.1 und 47.2 unterteilte Stange 47 mit dem daran angeformten Kopfstück 50 dargestellt. Die einzelnen Elemente der Stange 47 und des Kopfstücks 50 sind weitgehend analog dem vorstehend in Verbindung mit Fig.13 und 14 beschriebenen Ausführungsbeispiel ausgebildet. Abweichend davon sind bei dieser Variante die in den beiden Armen 47.1 und 47.2 vorgesehenen Aussparungen 55.1 und 55.2 sowie die beiden Wände 53 und 53.1 mit den Schenkeln 54 und 54.1 etwa halbkreisförmig ausgebildet. In der geschlossenen Stellung (Fig.16) sind die beiden stirnseitigen Schenkel 54 und 54.1 an den einander zugewandten Enden (nicht bezeichnet) formschlüssig gegeneinander gedrückt. Bei dieser Variante entspricht der mit **M** bezeichnete lichte Durchmesser der Ausnehmung 55 etwa dem Aussendurchmesser von 1,0 mm der röhrchenförmigen Sonde 46.

In Fig.17 ist eine weitere Variante der beispielsweise an dem ophthalmologischen Instrument 25 (Fig.3) anschraubbaren Funktionseinheit 35 mit der Führungshülse 45 in räumlicher Ansicht dargestellt. Abweichend von dem in Fig.4 und Fig.5 dargestellten Ausführungsbeispiel ist bei dieser Variante an dem einen Ende der Sonde 46 ein erstes in der Führungshülse 45 gelagertes Rohrstück 36 und an dem anderen Ende ein zweites Rohrstück 38 der Sonde 46 befestigt. Die am vorderen Ende mit dem als Fangglied ausgebildeten Kopfstück 50 versehene Stange 47 ist koaxial in dem zweiten Rohrstück 38 gelagert und durchdringt exzentrisch die daran anschliessende und als Hohlnadel ausgebildete Sonde 46 sowie das erste Rohrstück 36. Das erste Rohrstück 36 mit der Sonde 46 und dem zweiten Rohrstück 38 bilden zusammen mit dem in Fig.4 und 5 dargestellten Stellglied 40 eine in axialer Richtung relativ zu dem Kopfstück 50 verschiebbare Einheit.

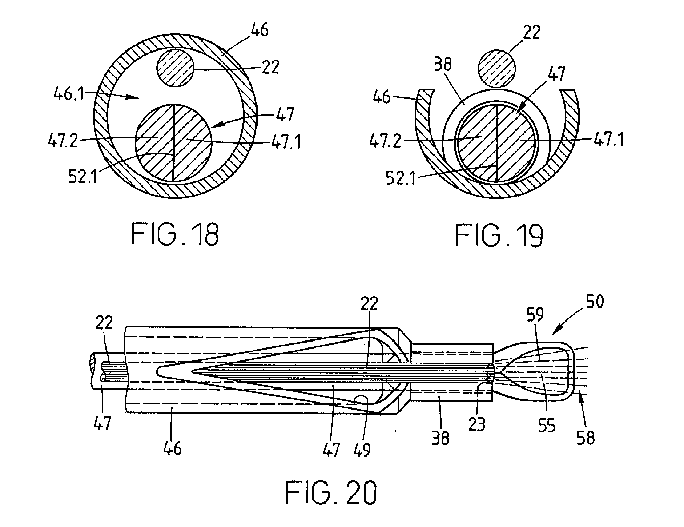

Im Bereich der Führungshülse 45 ist in dem ersten Rohrstück 36 zum Einführen eines Lichtleiters 22 eine entsprechend ausgebildete Eintrittsöffnung 37 vorgesehen, durch welche der Lichtleiter 22 in den Innenraum 46.1 (Fig.18) der Sonde 46 einführbar ist. Zum Herausführen des die Sonde 46 in axialer Richtung durchdringenden Lichtleiters 22 ist die Sonde 46 am anderen Ende mit einer Austrittsöffnung 49 versehen. Wie in Fig.17 schematisch dargestellt, ist der aus der Austrittsöffnung 49 herausgeführte Lichtleiter 22 derart aussenseitig an dem Rohrstück 38 angeordnet, dass das an der Stirnseite 23 austretende Licht die Ausnehmung 55 des Kopfstücks 50 beleuchtet.

Der Lichtleiter 22 steht mit einer in Fig.17 schematisch dargestellten Lichtquelle 21 in Verbindung. Die Lichtquelle 21 ist beispielsweise in Form einer Batterie in dem Gehäuse 24 des Instruments 25 (Fig.3) angeordnet. Bei einer weiteren Variante besteht die Möglichkeit, dass der Lichtleiter 21 direkt an eine nicht dargestellte ophthalmologische Einrichtung angeschlossen ist.

Fig.18 zeigt die in grösserem Massstab sowie gemäss der in Fig.17 eingezeichneten Linie D-D im Schnitt dargestellte Sonde 46 und man erkennt die exzentrisch im Innenraum 46.1 angeordnete Stange 47 mit den beiden im Profilquerschnitt dargestellten Armen 47.1 und 47.2 sowie den ebenfalls exzentrisch im Innenraum 46.1 angeordneten Lichtleiter 22.

Fig.19 zeigt das in grösserem Massstab sowie gemäss der in Fig.17 eingezeichneten Linie E-E im Schnitt dargestellte und an der Sonde 46 angeordnete zweite Rohrstück 38 mit der koaxial darin gelagerten Stange 47 sowie den am Aussendurchmesser des Rohrstücks 38 angeordneten Lichtleiter 22.

In Fig.20 ist das vordere Teilstück gemäss der in Fig.17 eingezeichneten Pfeilrichtung F in Draufsicht sowie in grösserem Massstab dargestellt und man erkennt die röhrchenförmige Sonde 46 mit der Austrittsöffnung 49 sowie das daran angeordnete zweite Rohrstück 38 mit dem in geschlossener Stellung dargestellten Kopfstück 50. Weiterhin erkennt man den aus der Austrittsöffnung 49 herausgeführten und am vorderen Rohrstück 38 angeordneten Lichtleiter 22. Der Lichtleiter 22 kann beispielsweise mit nicht dargestellten Mitteln an dem Rohrstück 38 befestigt werden. Der Lichtleiter 22 ist vorzugsweise mit einer in Bezug auf die nicht bezeichnete Längsachse desselben geneigt ausgebildeten Stirnseite 23 versehen, mittels welcher die einzelnen Strahlen 59 des abgestrahlten Lichtbündels 58 mit begrenztem Raumwinkel auf die im Kopfstück 50 vorgesehene Ausnehmung 55 gerichtet wird. Bei einem weiteren Ausführungsbeispiel ist entweder die Stirnseite 23 des Lichtleiters 22 als Linse ausgebildet oder an der Stirnseite 23 eine Linse angeordnet.

Wie in Fig.20 dargestellt, ist der aussenseitig an dem zweiten Rohrstück 38 angeordnete Lichtleiter 22 mit der Stirnseite 23 in Bezug auf die nicht bezeichnete Stirnseite des Rohrstücks 38 bündig abschliessend angeordnet.

An dieser Stelle wird darauf hingewiesen, dass die einzelnen vorstehend in Verbindung mit den Figuren 6 bis 16 beschriebenen Varianten der allgemein und jeweils mit 47 bezeichneten Stangen sowie die allgemein und jeweils mit 50 bezeichneten Kopfstücke auch analog bei dem Ausführungsbeispiel gemäss Fig.17 bis Fig.20 einsetzbar sind.

Die jeweils in der als Hohlnadel ausgebildeten Sonde 46 gelagerte Stange 47 ist vorzugsweise darart in dem Stellglied 40 angeordnet und mittels des Gewindestifts 43 (Fig.4,5) fixiert, dass die im Kopfstück 50 vorgesehene und zum Greifen und Halten des Blutgefässes 12 ausgebildete Ausnehmung 55, wie in Fig.2 schematisch dargestellt, für den Ophthalmologen gut sichtbar ist.

## Patentansprüche

1. Mikrochirurgisches Instrument, insbesondere ophthalmologisches Instrument (25) für die Netzhautchirurgie, bestehend aus einem als Handgriff ausgebildeten und mit Mitteln zum Betätigen eines Schiebebolzens (32) versehenen Gehäuse (24), einer daran angeordneten Funktionseinheit (35) mit darin gelagertem und mit dem Schiebebolzen (32) zusammenwirkenden Stellglied (40) sowie einer damit wirkverbundenen röhrchenförmigen Sonde (46), welche mindestens zur Aufnahme einer in axialer Richtung orientierten und am vorderen Ende mit einem Kopfstück versehenen Stange (47) ausgebildet ist, **dadurch gekennzeichnet, dass** die Stange (47) ausgehend von dem Kopfstück (50) zwei in axialer Richtung durch einen Spalt (52) getrennte und zur Erreichung einer federelastischen Vorspannung relativ zueinander spreizbare sowie am vorderen Ende jeweils mit einer Aussparung (55.1 und 55.2) versehene und entgegen der Vorspannung zusammendrückbare Arme (47.1 und 47.2) aufweist, bei welchen die einander zugewandten Aussparungen in der Schliessstellung eine quer zur Längsachse der Stange (47) orientierte und zum klemmfreien sowie freibeweglichen Halten feiner Strukturen ausgebildete Ausnehmung (55) bilden.

2. Mikrochirurgisches Instrument nach Anspruch 1, **dadurch gekennzeichnet, dass** dem aus der Sonde (46) herausragenden Kopfstück (50) ein in Richtung der Ausnehmung (55) orientierter und mit einer Lichtquelle (21) in Verbindung stehender Lichtleiter (22) zugeordnet ist.

3. Mikrochirurgisches Instrument nach Anspruch 2, **dadurch gekennzeichnet, dass** zur Erreichung eines in Richtung des Kopfstücks (50) abgestrahlten Lichtbündels (58) der Lichtleiter (22) mit einer die Ausnehmung (55) beleuchtenden Stirnseite (23) versehen ist.

4. Mikrochirurgisches Instrument nach Anspruch 2, **dadurch gekennzeichnet, dass** der in Richtung des Kopfstücks (50) orientierte Lichtleiter (22) an der der Ausnehmung (55) zugewandten Stirnseite (23) als konvexe Linse ausgebildet ist.

5. Mikrochirurgisches Instrument nach Anspruch 4, **dadurch gekennzeichnet, dass** an der Stirnseite (23) des Lichtleiters (22) eine Linse angeordnet ist.

6. Mikrochirurgisches Instrument nach einem der Ansprüche 3 bis 5, **dadurch gekennzeichnet, dass** die Stirnfläche (23) des Lichtleiters (22) in Bezug auf die Längsachse desselben in Richtung der am Kopfstück (50) angeordneten Ausnehmung (55) geneigt ausgebildet ist.

7. Mikrochirurgisches Instrument nach einem der Ansprüche 1 oder 2, **dadurch gekennzeichnet, dass** die an den beiden Armen (47.1 und 47.2) zu beiden Seiten der Symmetrieachse (S-S) vorgesehenen Aussparungen (55.1 und 55.2) durch stirnseitig angeformte Schenkel (54 und 54.1) begrenzt und derart klauenförmig ausgebildet sind, dass in geschlossenem Zustand des Kopfstücks (50) die beiden Schenkel (54 und 54.1) mittels einander zugewandter Kanten (56 und 56.1) formschlüssig gegeneinander drückbar sind.

8. Mikrochirurgisches Instrument nach Anspruch 7, **dadurch gekennzeichnet, dass** die einander zugewandten und als Stirnseite (60) des Kopfstücks (50) ausgebildeten Kanten (56 und 56.1) der beiden Schenkel (54 und 54.1) kleiner als der halbe Durchmesser der zylindrischen Stange (47) ausgebildet sind.

9. Mikrochirurgisches Instrument nach einem der Ansprüche 1 oder 2, **dadurch gekennzeichnet, dass** die an den beiden Armen (47.1 und 47.2) zu beiden Seiten der Symmetrieachse S-S vorgesehenen Aussparungen (55.1 und 55.2) jeweils ausgehend von dem stirnseitigen Schenkel (54 und 54.1) in Richtung des Spalts (52) etwa bogenförmig ausgebildet sind und in geschlossenem Zustand des Kopfstücks (50) eine etwa tropfenförmige Ausnehmung (55) bilden.

10. Mikrochirurgisches Instrument nach Anspruch 9, **dadurch gekennzeichnet, dass** die in axialer Richtung des Kopfstücks (50) orientierte lichte Länge (L) der tropfenförmigen Ausnehmung (55) grösser ist als die quer dazu orientierte lichte Breite (H).

11. Mikrochirurgisches Instrument nach einem der Ansprüche 1 oder 2, **dadurch gekennzeichnet, dass** die an den beiden Armen (47.1 und 47.2) zu beiden Seiten der Symmetrieachse (S-S) angeordneten Aussparungen (55.1 und 55.2) eine durch die stirnseitigen Schenkel (54 und 54.1) begrenzte und in geschlossenem Zustand des Kopfstücks (50) etwa länglich ausgebildete Ausnehmung (55) bilden.

12. Mikrochirurgisches Instrument nach Anspruch 11, **dadurch gekennzeichnet, dass** die in axialer Richtung des Kopfstücks (50) orientierte lichte Länge (L) der länglichen Ausnehmung (55) kleiner ist als die quer dazu orientierte lichte Breite (H).

13. Mikrochirurgisches Instrument nach einem der Ansprüche 1 oder 2, **dadurch gekennzeichnet, dass** die an den beiden Armen (47.1 und 47.2) zu beiden Seiten der Symmetrieachse (S-S) angeordneten Aussparungen (55.1 und 55.2) eine durch die stirnseitigen Schenkel (54 und 54.1) begrenzte und in geschlossenem Zustand des Kopfstücks (50) eine kreisförmige Ausnehmung (55) bilden.

14. Mikrochirurgisches Instrument nach Anspruch 13, **dadurch gekennzeichnet, dass** der lichte Durchmesser (M) der kreisförmigen Ausnehmung (55) etwa analog dem Aussendurchmesser der röhrchenförmigen Sonde (46) ausgebildet ist.

15. Mikrochirurgisches Instrument nach einem der Ansprüche 1 oder 2, **dadurch gekennzeichnet, dass** die beiden Arme (47.1 und 47.2) ausgehend von der zylindrischen Stange (47) in Richtung der Stirnseite (60) des Kopfstücks (50) konisch verjüngend und die beiden gegenüberliegenden Seitenwände (57 und 57.1) entweder geradlinig oder bogenförmig ausgebildet sind.

16. Mikrochirurgisches Instrument nach einem der Ansprüche 1 oder 2, **dadurch gekennzeichnet, dass** die zylindrische Stange (47) zwei im Profilquerschnitt halbkreisförmig ausgebildete und miteinander verbundene Teilstücke (48 und 48.1) umfasst, welche an dem einen Ende jeweils als klauenförmiges und mit der Ausnehmung (55.1 und 55.2) versehenes Kopfstück (50) ausgebildet sind.

17. Mikrochirurgisches Instrument nach einem der Ansprüche 1 oder 2, **dadurch gekennzeichnet, dass** die zur Aufnahme der Stange (47) und des Lichtleiters (22) röhrchenförmig ausgebildete Sonde (46) an dem einen Ende mit einem daran angeordneten ersten Rohrstück (36) in der Führungshülse (45) gelagert und an dem anderen Ende mit einem zur koaxialen Lagerung der Stange (47) ausgebildeten zweiten Rohrstück (38) versehen ist.

18. Mikrochirurgisches Instrument nach Anspruch 17, **dadurch gekennzeichnet, dass** die mit dem ersten Rohrstück (36) sowie mit dem zweiten Rohrstück (38) versehene Sonde (46) als eine Einheit relativ zu der feststehenden und am vorderen Ende mit dem Kopfstück (50) versehenen Stange (47) in axialer Richtung verschiebbar ist.

19. Mikrochirurgisches Instrument nach Anspruch 17, **dadurch gekennzeichnet, dass** zum Einführen des Lichtleiters (22) in dem ersten Rohrstück (36) eine Eintrittsöffnung (37) und in axialer Richtung im Abstand dazu in der Sonde (46) eine Austrittsöffnung (49) zum Herausführen des Lichtleiters (22) vorgesehen ist.

20. Mikrochirurgisches Instrument nach Anspruch 19, **dadurch gekennzeichnet, dass** der Lichtleiter (22) mit dem aus der Austrittsöffnung (49) der Sonde (46) herausragenden Ende aussenseitig an dem zweiten Rohrstück (38) angeordnet ist.
